# EUROPEAN PATENT APPLICATION

(11) **EP 0 889 127 A1**
(43) Date of publication of application: **07.01.1999**
(21) Application number: 98304830.7
(22) Date of filing: 18.06.1998
(51) Int. Cl.: C12N 15/12, C12N 15/63, C12N 1/11, C12N 1/13, C12N 1/15, C12N 1/19, C12N 1/21, C12N 5/10, C07K 16/00, C07K 14/47, C12Q 1/68, C12N 9/12

(54) **Serine/threonine protein kinase (H-SGK2)**

(30) Priority: 01.07.1997 US 51446 P; 23.12.1997 US 997212
(71) Applicant: SMITHKLINE BEECHAM CORPORATION, Philadelphia Pennsylvania 19103 (US)
(72) Inventor: Kumar, Sanjay, SmithKline Beecham Pharm., King of Prussia, Pennsylvania 19406 (US); Zou, Cheng, SmithKline Beecham Pharm., King of Prussia, Pennsylvania 19406 (US)
(74) Representative: Crump, Julian Richard John

(57) **Abstract**

H-SGK2 polypeptides and polynucleotides and methods for producing such polypeptides by recombinant techniques are disclosed. Also disclosed are methods for utilizing H-SGK2 polypeptides and polynucleotides in the design of protocols for the treatment of chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others, and diagnostic assays for such conditions.

## Description

This application claims the benefit of U.S. Provisional Application No.60/051,446, filed July 1, 1997.

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to serum glucocorticoid regulated kinase (SGK) family, hereinafter referred to as H-SGK2. The invention also relates to inhibiting or activating the action of such polynucleotides and polypeptides.

### BACKGROUND OF THE INVENTION

The serum glucocorticoid regulated kinase (sgk) was discovered in a rat mammary tumor cell line (Con8.hd6) to be a novel member of the serine/threonine protein kinase family whose expression is transcnptionally regulated by glucocorticoids and serum (Webster et al., 1993, Mol. Cell Biol. 13:2031-2041). It is expressed in other rat tissues such as the ovary, thymus and lung with lower levels in most other tissues (Webster et al., 1993, Mol. Cell Biol. 13:2031-2041). More recently, its human homologue, h-sgk, was cloned as a gene transcriptionally induced in HepG2 hepatocytes in response to changes in cellular hydration state (Waldegger et al., 1997 Proc. Natl. Acad. Sci., USA. 94:4440-4445). Sequence analysis of the full length rat and human sgk mRNA predicts a 431 amino acid protein of 49 kDa. The SGK protein contains a putative 270 amino acid catalytic domain made up of 11 distinct subdomains characteristic of serine/threonine protein kinases. The human and rat SGK proteins are 98% identical to each other and share sequence homology with other kinases in this region. These include rac protein kinase, protein kinase C, ribosomal protein S6 kinase and cyclic AMP dependent protein kinase. Despite the homology with other kinases, sgk has yet to be shown to be a functional kinase.

The promoter region of the rat sgk gene contains a functional glucocorticoid responsive element (GRE) and the induction of sgk appears to be a glucocorticoid receptor-specific response (Webster et al., 1993, Mol. Cell Biol. 13:2031-2041). A similar increase in sgk transcript levels is observed in rat mammary tumor cells after treatment with either dexamethasone, hydrocortisone or corticosterone, all of which use the glucocorticoid receptor pathway. Meanwhile, steroids which have an alternate means of gene activation, such as cholesterol, progesterone, b-estradiol, and testosterone, were unable to induce sgk mRNA expression (Webster et al., 1993, Mol. Cell Biol. 13:2031-2041). Serum also stimulates sgk mRNA expression in these cells although the pathway activation is unclear. Unlike rat sgk, the h-sgk expression in HepG2 cells is not regulated by either serum or glucocorticoid. Instead, the expression of h-sgk in HepG2 cells was increased by exposure of cells to hypertonic extracellular fluid and decreased by exposure to hypotonic media (Waldegger et al., 1997, Proc. Natl. Acad. Sci., USA. 94:4440-4445). Recently, p53, a tumor supressor protein, has been found to repress sgk promoter activity (Maiyar et al., 1997, Mol. Endocrin. 11:312-329). This represents the first example of a hormone regulated kinase gene regulated by p53 (Maiyar et al., 1996, J. Biol. Chem. 271:12414-12422).

The function of sgk in the cell is unclear. In some cell types sgk may play a role in cell growth. In the Con8.hd6 cells, glucocorticoid treatment, which increases sgk expression, decreases the proliferation. However, in other cell lines dexamethasone increases sgk levels without affecting cell growth. Some data suggests that it may play a role in mitogenic response in the G0-G1 transition in quiescent Rat2 fibroblasts since it is induced in response to serum stimulation and has a rapid half life like other immediate-early response genes (Webster et al., 1993, J. Biol. Chem. 268:11482-11485). One group proposes that sgk is involved in wound healing and regeneration in the central nervous system. Rat sgk gene was found to be strongly expressed in glial cells and oligodendrocytes surrounding lesions in the injured rat brain (Imaizumi et al., 1994, Mol. Brain Res. 26:189-196). Whatever its role, sgk are most likely involved in a complex series of phosphorlyation/dephosphorlyation events regulated separately and/or concurrently by a number of extracellular and intracellular factors such as glucocorticoids, serum, growth factors, cell volume, osmolarity and p53. A novel member of SGK family of kinases has been cloned.

This indicates that the SGK family has an established, proven history as therapeutic targets. Clearly there is a need for identification and characterization of further members of SGK family which can play a role in preventing, ameliorating or correcting dysfunctions or diseases, including, but not limited to, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease.

### SUMMARY OF THE INVENTION

In one aspect, the invention relates to H-SGK2 polypeptides and recombinant materials and methods for their production. Another aspect of the invention relates to methods for using such H-SGK2 polypeptides and polynucleotides. Such uses include the treatment of chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others. In still another aspect, the invention relates to methods to identify agonists and antagonists using the materials provided by the invention, and treating conditions associated withH-SGK2 imbalance with the identified compounds. Yet another aspect of the invention relates to diagnostic assays for detecting diseases associated with inappropriate H-SGK2 activity or levels.

### DESCRIPTION OF THE INVENTION

### Definitions

The following definitions are provided to facilitate understanding of certain terms used frequently herein.

"H-SGK2" refers, among others, generally to a polypeptide having the amino acid sequence set forth in SEQ ID NO:2 or an allelic variant thereof.

"H-SGK2 activity or H-SGK2 polypeptide activity" or "biological activity of the H-SGK2 or H-SGK2 polypeptide" refers to the metabolic or physiologic function of said H-SGK2 including similar activities or improved activities or these activities with decreased undesirable side-effects. Also included are antigenic and immunogenic activities of saidH-SGK2.

"H-SGK2 gene" refers to a polynucleotide having the nucleotide sequence set forth in SEQ ID NO: 1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter et *al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptidethat differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Canllo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to H-SGK2 polypeptides (or H-SGK2 proteins) . The H-SGK2 polypeptides include the polypeptide of SEQ ID NOS:2 and 4; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included withinH-SGK2 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. PreferablyH-SGK2 polypeptide exhibit at least one biological activity ofH-SGK2.

The H-SGK2 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the H-SGK2 polypeptides are also included in the invention. A fragment is a polypeptide having an amino acid sequence that entirely is the same as part, but not all, of the amino acid sequence of the aforementioned H-SGK2 polypeptides. As with H-SGK2 polypeptides, fragments may be "free-standing," or compnsed within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of H-SGK2 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of H-SGK2 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate H-SGK2 activity, including those with a similar activity or an improved activity, or with a decreased undesirable activity. Also included are those that are antigenic or immunogenic in an animal, especially in a human.

Preferably, all of these polypeptide fragments retain the biological activity of the H-SGK2, including antigenic activity. Among the most preferred fragment is that having the amino acid sequence of SEQ ID NO: 4. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The H-SGK2 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to H-SGK2 polynucleotides. H-SGK2 polynucleotides include isolated polynucleotides which encode the H.-SGK2 polypeptides and fragments, and polynucleotides closely related thereto. More specifically, H-SGK2 polynucleotide of the invention include a polynucleotide comprising the nucleotide sequence contained in SEQ ID NO:1 encoding a H-SGK2 polypeptide of SEQ ID NO: 2, and polynucleotides having the particular sequences of SEQ ID NOS:1 and 3. H-SGK2 polynucleotides further include a polynucleotide comprising a nucleotide sequence that has at least 80% identity over its entire length to a nucleotide sequence encoding the H-SGK2 polypeptide of SEQ ID NO:2, and a polynucleotide comprising a nucleotide sequence that is at least 80% identical to that of SEQ ID NO: 1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under H-SGK2 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to suchH-SGK2 polynucleotides.

H-SGK2 of the invention is structurally related to other proteins of the SGK family, as shown by the results of sequencing the cDNA encoding human H-SGK2. The cDNA sequence of SEQ ID NO:1 contains an open reading frame (nucleotide number 129 to 1616) encoding a polypeptide of 496 amino acids of SEQ ID NO:2. The amino acid sequence of Table 2 (SEQ ID NO:2) has about 71.2 % identity (using BLAST) in 389 amino acid residues with rat serum and glucorticoid-regulated kinase (sgk, GENBANK Accession # L01624, M.K. Webster et al., Mol. Cell Biol. 13:2031-2041, 1993). Furthermore, H-SGK2 (SEQ ID NO:2) is 70.7 % identical to human serine/threonine protein kinase (H-SGK, GENBANK Accesion# Y10032, S. Waldegger et al., Proc. Natl. Acad. Sci., USA. 94:4440-4445, 1997) over 389 amino acid residues. The nucleotide sequence of Table 1 (SEQ ID NO:1) has about 68.57 % identity (using BLAST) in 1050 nucleotide residues with human serine/threonine protein kinase (H-SGK, GENBANK Accesion# Y10032, S. Waldegger et al., Proc. Natl. Acad. Sci., USA. 94:4440-4445, 1997). Furthermore, H-SGK2 (SEQ ID NO:1) is 67.7 % identical to rat serum and glucorticoid-regulated kinase (SGK, GENBANK Accession # L01624) over 1030 nucleotide residues (M.K. Webster et al., Mol. Cell Biol. 13:2031-2041, 1993). Thus, H-SGK2 polypeptides and polynucleotides of the present invention are expected to have, inter alia, similar biological functions/properties to their homologous polypeptides and polynucleotides, and their utility is obvious to anyone skilled in the art.

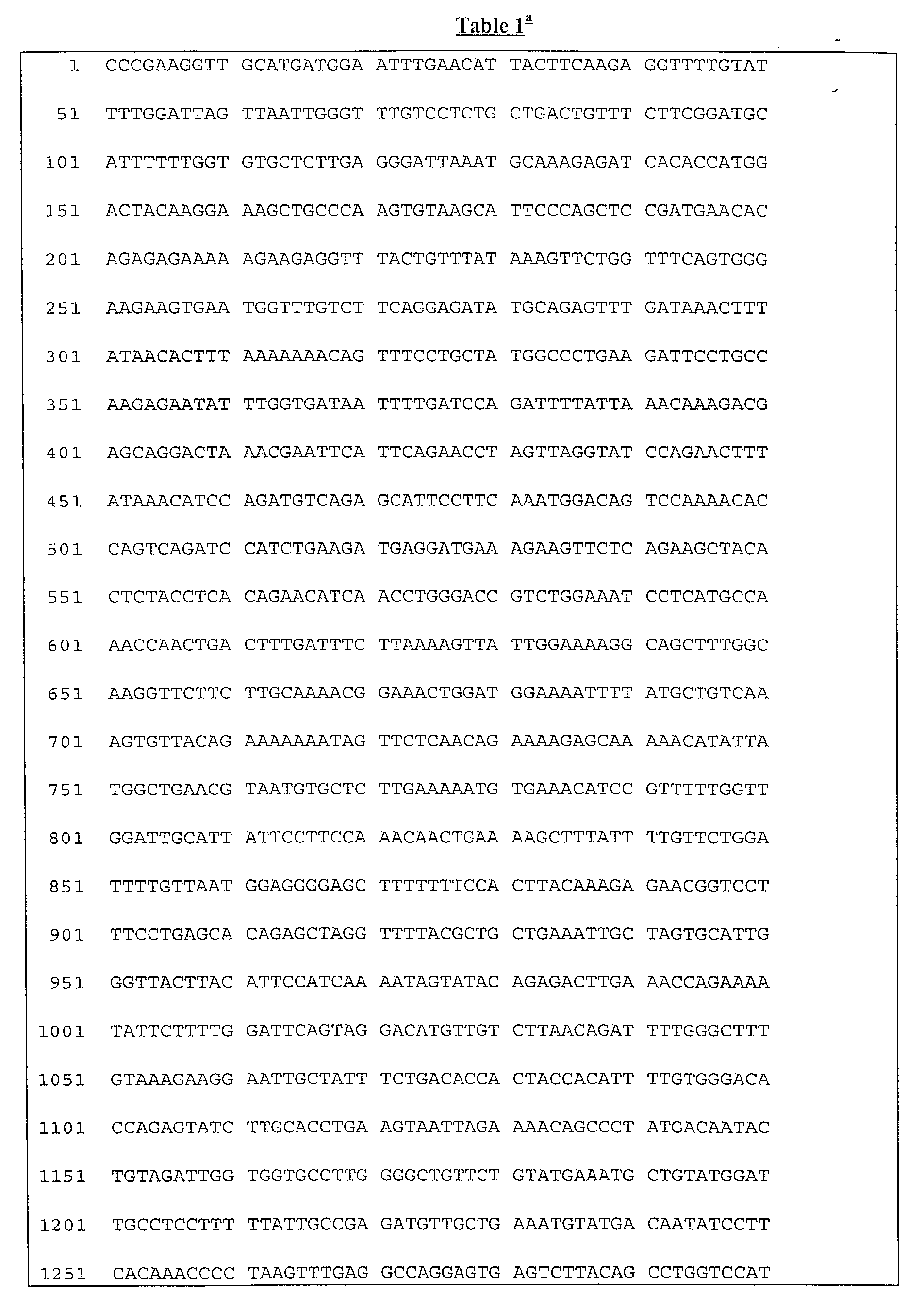

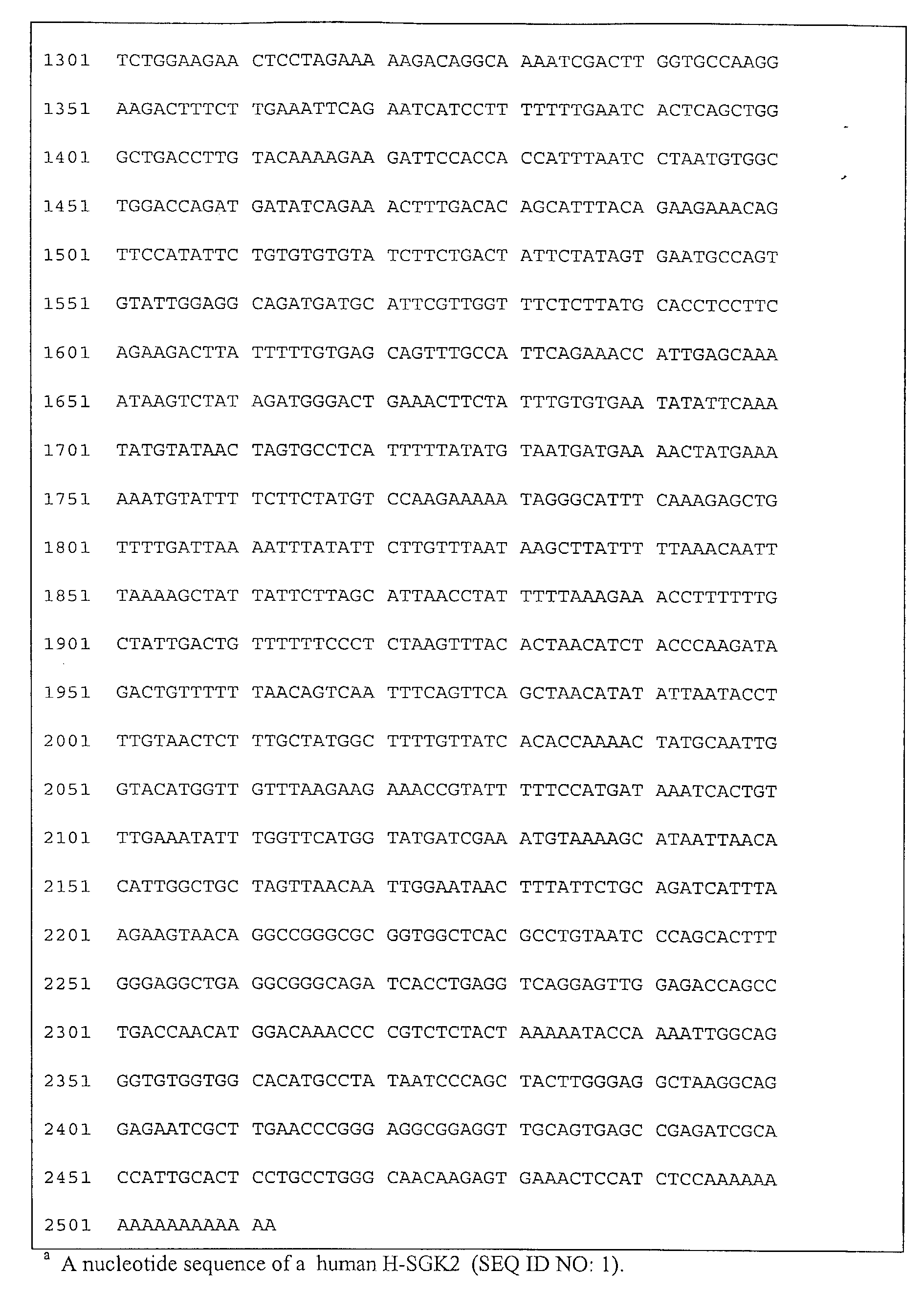

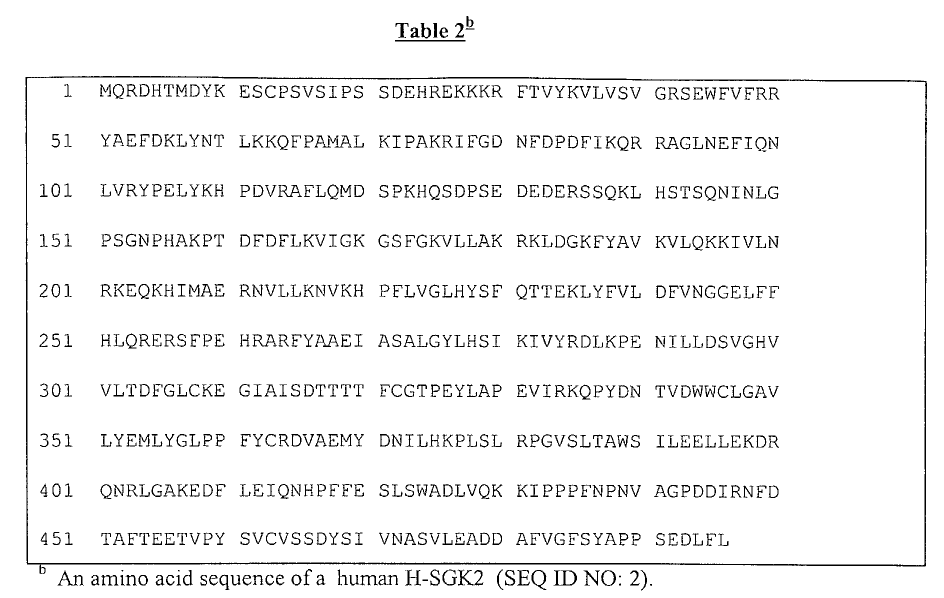

One polynucleotide of the present invention encoding H-SGK2 may be obtained using standard cloning and screening, from a cDNA library derived from mRNA in cells of human brain (hippocampus), primary osteoblasts and primary dendritic cells using the expressed sequence tag (EST) analysis (Adams, M.D., *el al. Science* (1991) 252:1651-1656; Adams, M.D. *et al., Nature,* (1992) 355:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding H-SGK2 polypeptide of SEQ ID NO:2 may be identical to the polypeptide encoding sequence contained in Table 1 (nucleotide number 129 to 1616 of SEQ ID NO: 1), or it may be a sequence, which as a result of the redundancy (degeneracy) of the genetic code, also encodes the polypeptide of SEQ ID NO:2.

When the polynucleotides of the invention are used for the recombinant production ofH-SGK2 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding H-SGK2 variants comprising the amino acid sequence of H-SGK2 polypeptide of Table 2 (SEQ ID NO:2) in which several, 5-10, 1-5, 1-3, 1-2 or 1 amino acid residues are substituted, deleted or added, in any combination. Among the preferred polynucleotides of the present invention is contained in Table 3 (SEQ ID NO: 3) encoding the amino acid sequence of Table 4 (SEQ ID NO: 4).

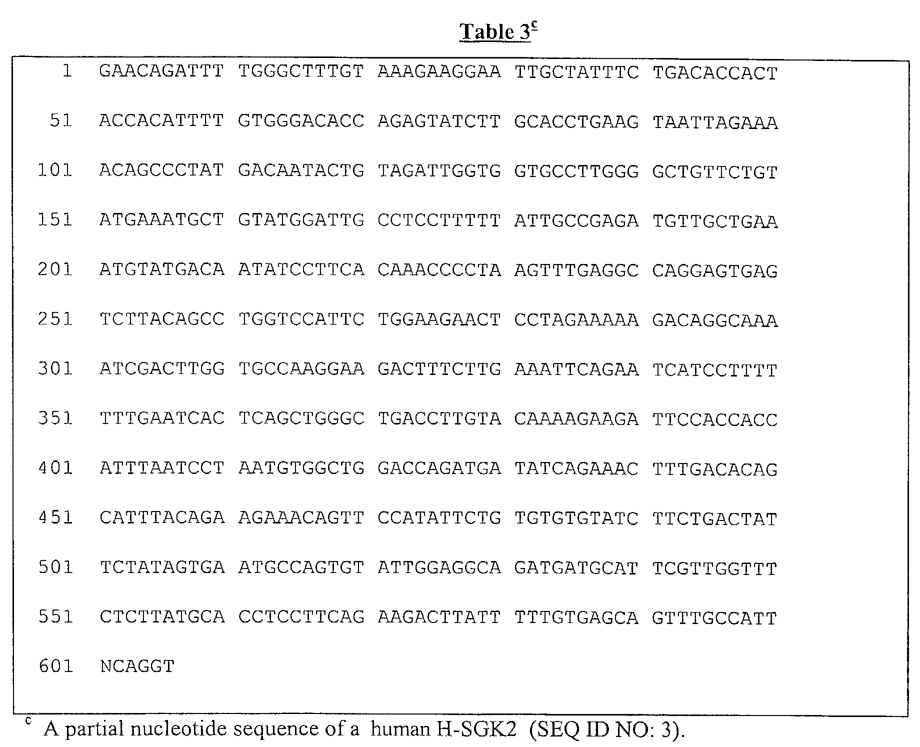

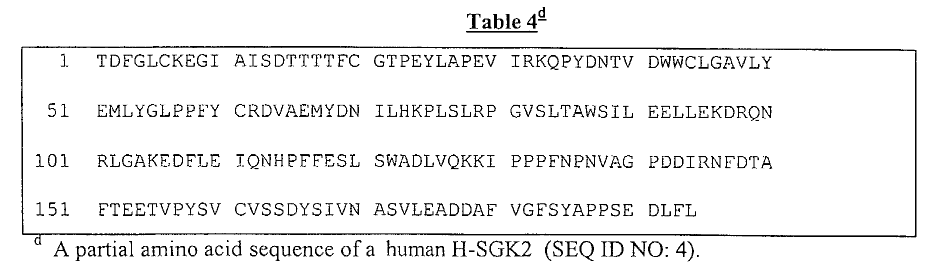

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO:1 or a fragment thereof (including that of SEQ ID NO:3), may be used as hybridization probes for cDNA and genomic DNA, to isolate full-length cDNAs and genomic clones encoding H-SGK2 polypeptide and to isolate cDNA and genomic clones of other genes (including genes encoding homologs and orthologs from species other than human) that have a high sequence similarity to the H-SGK2 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding H-SGK2 polypeptide, including homologs and orthologs from species other than human, comprises the steps of screening an appropriate library under stingent hybridization conditions with a labeled probe having the SEQ ID NO: 1 or a fragment thereof (including that of SEQ ID NO: 3), and isolating full-length cDNA and genomic clones containing said polynucleotide sequence. Such hybridization techniques are well known to those of skill in the art. Thus in another aspect, H-SGK2 polynucleotides of the present invention further include a nucleotide sequence comprising a nucleotide sequence that hybndize under stringent condition to a nucleotide sequence having SEQ ID NO: 1 or a fragment thereof (including that of SEQ ID NO:3). Also included with H-SGK2 polypeptides are polypeptide compnsing amino acid sequence encoded by nucleotide sequence obtained by the above hybridization condition. Stringent hybridization conditions are as defined above or, alternatively, conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods descnbed in many standard laboratory manuals, such as Davis et al., *BASIC METHODS IN MOLECULAR BIOLOGY* (1986) and Sambrook et al., *MOLECULAR CLONING. A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors denved from combinations thereof, such as those derived from plasmid and bactenophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL (supra).*

For secretion of the translated p:rotein into the lumen of the endoplasmic reticulum, into the periplasmic space or into the extracellular environment, appropnate secretion signals may be incorporated into the desired polypeptide. These signals may be endogenous to the polypeptide or they may be heterologous signals.

If the H-SGK2 polypeptide is to be expressed for use in screening assays, generally, it is preferred that the polypeptide be produced at the surface of the cell. In this event, the cells may be harvested prior to use in the screening assay. IfH-SGK2 polypeptide is secreted into the medium, the medium can be recovered in order to recover and purify the polypeptide; if produced intracellularly, the cells must first be lysed before the polypeptide is recovered.
H-SGK2 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of H-SGK2 polynucleotides for use as diagnostic reagents. Detection of a mutated form of H-SGK2 gene associated with a dysfunction will provide a diagnostic tool that can add to or define a diagnosis of a disease or susceptibility to a disease which results from under-expression, over-expression or altered expression of H-SGK2. Individuals carrying mutations in the H-SGK2 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled H-SGK2 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising H-SGK2 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease through detection of mutation in the H-SGK2 gene by the methods described.

In addition, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, can be diagnosed by methods comprising determining from a sample derived from a subject an abnormally decreased or increased level ofH-SGK2 polypeptide or H-SGK2 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an H-SGK2 polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, which comprises:
(a) a H-SGK2 polynucleotide, preferably the nucleotide sequence of SEQ ID NO: 1, or a fragment thereof;
(b) a nucleotide sequence complementary to that of (a);
(c) a H-SGK2 polypeptide, preferably the polypeptide of SEQ ID NO: 2, or a fragment thereof; or
(d) an antibody to a H-SGK2 polypeptide, preferably to the polypeptide of SEQ ID NO: 2.
It will be appreciated that in any such kit, (a), (b), (c) or (d) may comprise a substantial component.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies Immunospecific for the H-SGK2 polypeptides. The term "immunospecific" means that the antibodies have substantiall greater affinity for the polypeptides of the invention than their affinity for other related polypeptides in the prior art.

Antibodies generated against the H-SGK2 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybndoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against H-SGK2 polypeptides may also be employed to treat chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others.

### Vaccines

Another aspect of the invention relates to a method for inducing an immunological response in a mammal which comprises inoculating the mammal withH-SGK2 polypeptide, or a fragment thereof, adequate to produce antibody and/or T cell immune response to protect said animal from chronic and acute inflammation, arthritis, osteoarthritis, septicemia, autoimmune diseases (e.g., inflammatory bowel disease, psonasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others. Yet another aspect of the invention relates to a method of inducing immunological response in a mammal which comprises, deliveringH-SGK2 polypeptide via a vector directing expression ofH-SGK2 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a H-SGK2 polypeptide wherein the composition comprises aH-SGK2 polypeptide or H-SGK2 gene. The vaccine formulation may further comprise a suitable carrier. SinceH-SGK2 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multidose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The H-SGK2 polypeptide of the present invention may be employed in a screening process for compounds which activate (agonists) or inhibit activation of (antagonists, or otherwise called inhibitors) the H-SGK2 polypeptide of the present invention. Thus, polypeptides of the invention may also be used to assess identify agonist or antagonists from, for example, cells, cell-free preparations, chemical libranes, and natural product mixtures. These agonists or antagonists may be natural or modified substrates, ligands, enzymes, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

H-SGK2 polypeptides are responsible for many biological functions, including many pathologies. Accordingly, it is desirous to find compounds and drugs which stimulate H-SGK2 polypeptide on the one hand and which can inhibit the function of H-SGK2 polypeptide on the other hand. In general, agonists are employed for therapeutic and prophylactic purposes for such conditions as chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease. Antagonists may be employed for a variety of therapeutic and prophylactic purposes for such conditions as chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease.

In general, such screening procedures may involve using appropriate cells which express the H-SGK2 polypeptide or respond to H-SGK2 polypeptide of the present invention. Such cells include cells from mammals, yeast, *Drosophila* or *E. coli.* Cells which express the H-SGK2 polypeptide (or cell membrane containing the expressed polypeptide) or respond to H-SGK2 polypeptide are then contacted with a test compound to observe binding, or stimulation or inhibition of a functional response. The ability of the cells which were contacted with the candidate compounds is compared with the same cells which were not contacted for H-SGK2 activity.

The knowledge that the H-SGK2 may encode a protein kinase suggests that recombinant forms can be used to establish a protein kinase activity. Typically this would involve the direct incubation of H-SGK2 with a protein or peptide substrate in the presence of ³²p-γ-ATP, followed by the measurement of radioactivity incorporated into the substrate by separation and counting. Separation methods include immunoprecipitation, conjugation of substrate to a bead allowing separation by centrifugation or determination of incorporation by scintillation proximity assay, SDS-PAGE followed by autoradiography or biosensor analysis. While the specific substrates are not yet known, candidates include H-SGK2 itself (autophosphorylation), myelin basic protein, casein, histone and HSP27. Other substrates might be discovered by incubating H-SGK2 with random peptides conjugated to solid supports or displayed on the surface of phage or by incubation of H-SGK2 with mammalian cell lysates and ³²P-γ-ATP, followed by separation of the labeled target proteins. and sequencing. The protein kinase activity of H-SGK2 may require incubation with a specific upstream effector. This may be achieved by preincubating H-SGK2 with lysates from a variety of stimulated eukaryotic cells and -³²P-γ-ATP.

This invention contemplates the treatment and/or amelioration of such diseases by administering an H-SGK2 inhibiting amount of a compound. Although the functioning of the H-SGK2 of this invention has not been conclusively established, it is believed that among the useful inhibitors of H-SGK2 function are those compounds which inhibit the kinase activity of the H-SGK2. Other sites of inhibition are, of course, possible owing to its position in a signal transduction cascade. Therefore, inhibiting the interaction of H-SGK2 with one or more of its upstream or downstream modulators/substrates is also contemplated by this invention. Inhibitors of protein-protein interactions between H-SGK2 and other factors could lead to the development of pharmaceutical agents for the modulation of H-SGK2 activity.

In one specific embodiment, a method to identify possible inhibitors of the H-SGK2 is described here. The peptide substrate of H-SGK2 will be biotinlyated at one end. It will then be added to a 96 well streptavidin coated flash plate. The high affinity binding of the biotin to streptavidin in the wells will anchor the peptide in the well and allow the residues to be accessible for phosphorylation. Purified active H-SGK2, ³³P-γ-ATP and compounds will then be incubated with the peptide at a suitable temperature for kinase activity to occur. The plates will be washed and the amount of phosphorlyated peptide will be determined. Those wells with the least amount of radioactivity could contain a possible inhibitor.

### H-SGK2 binding molecules and assays

H-SGK2 could be used to isolate proteins which interact with it and this interaction could be a target for interference. Inhibitors of protein-protein interactions between H-SGK2 and other factors could lead to the development of pharmaceutical agents for the modulation of H-SGK2 activity. As used herein, the term "modulate" refers to affecting the H-SGK2 function.

Thus, this invention also provides a method for identification of binding molecules to H-SGK2. Genes encoding proteins for binding molecules to H-SGK2 can be identified by numerous methods known to those of skill in the art, for example, ligand panning and FACS sorting. Such methods are descnbed in many laboratory manuals such as, for instance, Coligan et al., Current Protocols in Immunology 1 (Rivett, A.J. Biochem. J. 291:1-10(1993)): Chapter 5 (1991).

For example, the yeast two-hybrid system provides methods for detecting the interaction between a first test protein and a second test protein, in vivo, using reconstitution of the activity of a transcriptional activator. The method is disclosed in U.S. Patent No. 5,283,173; reagents are available from Clontech and Stratagene. Briefly, H-SGK2 cDNA is fused to a Gal4 transcription factor DNA binding domain and expressed in yeast cells. cDNA library members obtained from cells of interest are fused to a transactivation domain of Gal4. cDNA clones which express proteins which can interact with H-SGK2 will lead to reconstitution of Gal4 activity and transactivation of expression of a reporter gene such as Gal1-lacZ.

An alternative method is screening of lambda gtl1, ZAP (Stratagene) or equivalent cDNA expression libraries with recombinant H-SGK2. Recombinant H-SGK2 protein or fragments thereof are fused to small peptide tags such as FLAG, HSV or GST. The peptide tags can possess convenient phosphorylation sites for a kinase such as heart muscle creatine kinase or they can be biotinylated. Recombinant H-SGK2 can be phosphorylated with -33p-γ -ATP or used unlabeled and detected with streptavidin or antibodies against the tags. gtl 1cDNA expression libraries are made from cells of interest and are incubated with the recombinant H-SGK2, washed and cDNA clones isolated which interact with H-SGK2. See, e.g., T. Maniatis et al, supra.

Another method is the screening of a mammalian expression library in which the cDNAs are cloned into a vector between a mammalian promoter and polyadenylation site and transiently transfected in COS or 293 cells followed by detection of the binding protein 48 hours later by incubation of fixed and washed cells with a labelled H-SGK2, preferably iodinated, and detection of bound H-SGK2 by autoradiography. See Sims et al., Science 241:585-589 (1988) and McMahan et al., EMBO J. 10:2821-2832 (1991). In this manner, pools of cDNAs containing the cDNA encoding the binding protein of interest can be selected and the cDNA of interest can be isolated by further subdivision of each pool followed by cycles of transient transfection, binding and autoradiography. Alternatively, the cDNA of interest can be isolated by transfecting the entire cDNA library into mammalian cells and panning the cells on a dish containing H-SGK2 bound to the plate. Cells which attach after washing are lysed and the plasmid DNA isolated, amplified in bacteria, and the cycle of transfection and panning repeated until a single cDNA clone is obtained. See Seed et al, Proc. Natl. Acad. Sci. USA 84:3365 (1987) and Aruffo et al., EMBO J. 6:3313 (1987). If the binding protein is secreted, its cDNA can be obtained by a similar pooling strategy once a binding or neutralizing assay has been established for assaying supernatants from transiently transfected cells. General methods for screening supernatants are disclosed in Wong et al., Science 228:810-815 (1985).

Another alternative method is isolation of proteins interacting with H-SGK2 directly from cells. Fusion proteins of H-SGK2 with GST or small peptide tags are made and immobilized on beads. Biosynthetically labeled or unlabeled protein extracts from the cells of interest are prepared, incubated with the beads and washed with buffer. Proteins interacting with H-SGK2 are eluted specifically from the beads and analyzed by SDS-PAGE. Binding partner primary amino acid sequence data are obtained by microsequencing. Optionally, the cells can be treated with agents that induce a functional response such as tyrosine phosphorylation of cellular proteins. An example of such an agent would be a growth factor, cellular stress or cytokine such as interleukin-2.

Another alternative method is immunoaffinity purification. Recombinant H-SGK2 is incubated with labeled or unlabeled cell extracts and immunoprecipitated with anti-H-SGK2 antibodies. The immunoprecipitate is recovered with protein A-Sepharose and analyzed by SDS-PAGE. Unlabeled proteins are labeled by biotinylation and detected on SDS gels with streptavidin. Binding partner proteins are analyzed by microsequencing. Further, standard biochemical purification steps known to those skilled in the art may be used prior to microsequencing.

Yet another alternative method is screening of peptide libraries for binding partners. Recombinant tagged or labeled H-SGK2 is used to select peptides from a peptide or phosphopeptide library which interact with H-SGK2. Sequencing of the peptides leads to identification of consensus peptide sequences which might be found in interacting proteins.

H-SGK2 binding partners identified by any of these methods or other methods which would be known to those of ordinary skill in the art as well as those putative binding partners discussed above can be used in the assay method of the invention. Assaying for the presence of H-SGK2/binding partner complex are accomplished by, for example, the yeast two-hybrid system, ELISA or immunoassays using antibodies specific for the complex. In the presence of test substances (i.e. inhibitors or antagonists) which interrupt or inhibit formation of H-SGK2/binding partner interaction, a decreased amount of complex will be determined relative to a control lacking the test substance.

Assays for free H-SGK2 or binding partner are accomplished by, for example, ELISA or immunoassay using specific antibodies or by incubation of radiolabeled H-SGK2 with cells or cell membranes followed by centrifugation or filter separation steps. In the presence of test substances which interrupt or inhibit formation of H-SGK2/binding partner interaction (i.e. inhibitors or antagonists), an increased amount of free human sgk2 or free binding partner will be determined relative to a control lacking the test substance.

Polypeptides of the invention also can be used to assess H-SGK2 binding capacity of H-SGK2 binding molecules in cells or in cell-free preparations.

The assays may simply test binding of a candidate compound wherein adherence to the cells bearing the H-SGK2 polypeptide is detected by means of a label directly or indirectly associated with the candidate compound or in an assay involving competition with a labeled competitor. Further, these assays may test whether the candidate compound results in a signal generated by activation of the H-SGK2 polypeptide, using detection systems appropriate to the cells bearing theH-SGK2 polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a H-SGK2 polypeptide to form a mixture, measuringH-SGK2 activity in the mixture, and comparing the H-SGK2 activity of the mixture to a standard.

The H-SGK2 cDNA, protein and antibodies to the protein may also be used to configure assays for detecting the effect of added compounds on the production of H-SGK2 mRNA and protein in cells. For example, an ELISA may be constructed for measuring secreted or cell associated levels of H-SGK2 protein using monoclonal and polyclonal antibodies by standard methods known in the art, and this can be used to discover agents which may inhibit or enhance the production of H-SGK2 (also called antagonist or agonist, respectively) from suitably manipulated cells or tissues.

The H-SGK2 protein may be used to identify membrane bound or soluble receptors, if any, through standard receptor binding techniques known in the art. These include, but are not limited to, ligand binding and crosslinking assays in which the H-SGK2 is labeled with a radioactive isotope (e.g., 125I), chemically modified (e.g., biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of H-SGK2 which compete with the binding of H-SGK2 to its receptors, if any. Standard methods for conducting screening assays are well understood in the art.

Examples of potential H-SGK2 polypeptide antagonists include antibodies or, in some cases, oligonucleotides or proteins which are closely related to the ligands, substrates, enzymes, receptors, etc., as the case may be, of the H-SGK2 polypeptide, e.g., a fragment of the ligands, substrates, enzymes, receptors, etc.; or small molecules which bind to the polypeptide of the present invention but do not elicit a response, so that the activity of the polypeptide is prevented.

Thus in another aspect, the present invention relates to a screening kit for identifying agonists, antagonists, ligands, receptors, substrates, enzymes, etc. forH-SGK2 polypeptides; or compounds which decrease or enhance the production of H-SGK2 polypeptides, which comprises:
(a) a H-SGK2 polypeptide, preferably that of SEQ ID NO:2;
(b) a recombinant cell expressing a H-SGK2 polypeptide, preferably that of SEQ ID NO:2;
(c) a cell membrane expressing a H-SGK2 polypeptide; preferably that of SEQ ID NO: 2; or
(d) antibody to a H-SGK2 polypeptide, preferably that of SEQ ID NO: 2.
It will be appreciated that in any such kit, (a), (b), (c) or (d) may comprise a substantial component.

### Prophylactic and Therapeutic Methods

This invention provides methods of treating abnormal conditions such as, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, related to both an excess of and insufficient amounts of H-SGK2 polypeptide activity.

If the activity of H-SGK2 polypeptide is in excess, several approaches are available. One approach comprises administering to a subject an inhibitor compound (antagonist) as hereinabove described along with a pharmaceutically acceptable carrier in an amount effective to inhibit the function of the H-SGK2 polypeptide, such as, for example, by blocking the binding of ligands, substrates, enzymes, receptors, etc., or by inhibiting a second signal, and thereby alleviating the abnormal condition. In another approach, soluble forms of H-SGK2 polypeptides still capable of binding the ligand, substrate, enzymes, receptors, etc. in competition with endogenousH-SGK2 polypeptide may be administered. Typical embodiments of such competitors comprise fragments of theH-SGK2 polypeptide.

In another approach, soluble forms ofH-SGK2 polypeptides still capable of binding the ligand in competition with endogenous H-SGK2 polypeptide may be administered. Typical embodiments of such competitors comprise fragments of the H-SGK2 polypeptide.

In still another approach, expression of the gene encoding endogenous H-SGK2 polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science (1991)* 251:1360. These oligomers can be administered *per se* or the relevant oligomers can be expressed *in vivo.*

For treating abnormal conditions related to an under-expression of H-SGK2 and its activity, several approaches are also available. One approach comprises administering to a subject a therapeutically effective amount of a compound which activates H-SGK2 polypeptide, i.e., an agonist as described above, in combination with a pharmaceutically acceptable carrier, to thereby alleviate the abnormal condition. Alternatively, gene therapy may be employed to effect the endogenous production of H-SGK2 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of H-SGK2 polypeptides in combination with a suitable pharmaceutical carrier.

### Formulation and Administration

Peptides, such as the soluble form of H-SGK2 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such camers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

### Example:

Two partial clones (EST # 2138008 and EST # 1629570) encoding a putative serine/threonine protein kinase were identified through a search of a commercial EST database. Both clones were completely sequenced on both strands and the sequence of these two ESTs were combined into an assembly using a commercial sequence analysis software. The resulting sequence did not contain an initiating methionine codon and, therefore, was not full length. Based on the most 5' sequence of available partial H-SGK2 cDNA sequence, three gene specific primers (primers A, B and C) were synthesized. Human Brain (Hippocampus) Marathon-Ready cDNA kit (Clontech, laboratories, Inc. Palo Alto, CA) was used to isolate the 5' end of H-SGK2 by PCR. First, the adaptor primer 1 (AP1, Clontech) and H-SGK2 primer A (5'- TGT GGT AGT GGT GTC AGA ATA GCA ATT CC -3') (SEQ ID NO:5) were used for 5'-RACE (rapid amplification of cDNA ends) reaction. The reaction mixture thus generated was used in two nested PCR as a template. Adaptor primer 2 (AP2, Clontech) and H-SGK2 primer B (5'- AAC CTA GCT CTG TGC TCA GGA AAG GAC -3') (SEQ ID NO:6) were used in one nested PCR whereas adapter primer 2 (AP2) and H-SGK2 primer C (5'- TTC CAT CCC AGT TTC CGT TTT GCA AGA A -3') (SEQ ID NO:7) were used in the other nested PCR. The PCR amplified 5' ends of H-SGK2 cDNA from both nested PCRs were then subcloned into PCR2.1 (Invitrogen, Inc. of San Diego, CA) vector and sequenced on both strands. The full sequence of this gene shares 68 % identity over 1050 nucleotides and 71 % identity over 389 amino acid residues with h-sgk (Waldegger et al., 1997 Proc. Natl. Acad. Sci., USA. 94:4440-4445). Therefore, this gene was named H-SGK2. The clone encoding H-SGK2 was found in a human brain (hippocampus), primary osteoblasts and primary human dendritic cell cDNA libraries.

All publications, including but not limited to patents and patent applications, cited in this specification are herein incorporated by reference as if each individual publication were specifically and individually indicated to be incorporated by reference herein as though fully set forth.

## Claims

1. An isolated polynucleotide comprising a nucleotide sequence that has at least 80% identity over its entire length to a nucleotide sequence encoding the H-SGK2 polypeptide of SEQ ID NO:2; or a nucleotide sequence complementary to said isolated polynucleotide.

2. The polynucleotide of claim 1 wherein said polynucleotide comprises the nucleotide sequence contained in SEQ ID NO: 1 encoding the H-SGK2 polypeptide of SEQ ID NO2.

3. The polynucleotide of claim 1 wherein said polynucleotide comprises a nucleotide sequence that is at least 80% identical to that of SEQ ID NO: 1 over its entire length.

4. The polynucleotide of claim 3 which is polynucleotide of SEQ ID NO: 1.

5. The polynucleotide of claim 1 which is DNA or RNA.

6. A DNA or RNA molecule comprising an expression system, wherein said expression system is capable of producing a H-SGK2 polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a H-SGK2 polypeptide comprising culturing a host of claim 7 under conditions sufficient for the production of said polypeptide and recovering the polypeptide from the culture.

9. A process for producing a cell which produces a H-SGK2 polypeptide thereof comprising transforming or transfecting a host cell with the expression system of claim 6 such that the host cell, under appropriate culture conditions, produces aH-SGK2 polypeptide.

10. A H-SGK2 polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the H-SGK2 polypeptide of claim 10.

13. A method for the treatment of a subject in need of enhanced activity or expression of H-SGK2 polypeptide of claim 10 comprising:
(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the H-SGK2 polypeptide of SEQ ID NO:2 over its entire length; or a nucleotide sequence complementary to said nucleotide sequence in a form so as to effect production of said polypeptide activity *in vivo.*

14. A method for the treatment of a subject having need to inhibit activity or expression of H-SGK2 polypeptide of claim 10 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

15. A process for diagnosing a disease or a susceptibility to a disease in a subject related to expression or activity ofH-SGK2 polypeptide of claim 10 in a subject comprising:
(a) determining the presence or absence of a mutation in the nucleotide sequence encoding said H-SGK2 polypeptide in the genome of said subject; and/or
(b) analyzing for the presence or amount of the H-SGK2 polypeptide expression in a sample derived from said subject.

16. A method for identifying compounds which inhibit (antagonize) or agonize the H-SGK2 polypeptide of claim 10 which comprises:
(a) contacting a candidate compound with cells which express the H-SGK2 polypeptide (or cell membrane expressing H-SGK2 polypeptide) or respond to H-SGK2 polypeptide; and
(b) observing the binding, or stimulation or inhibition of a functional response; or comparing the ability of the cells (or cell membrane) which were contacted with the candidate compounds with the same cells which were not contacted for H-SGK2 polypeptide activity.

17. An agonist identified by the method of claim 16.

18. An antagonist identified by the method of claim 16.

19. A recombinant host cell produced by a method of Claim 9 or a membrane thereof expressing a H-SGK2 polypeptide.


### Annex to the description

SEQUENCE LISTING

(1) GENERAL INFORMATION

(i) APPLICANT: SmithKline Beecham Corporation

(ii) TITLE OF THE INVENTION: SERINE/THREONINE PROTEIN KINASE
(H-SGK2)

(iii) NUMBER OF SEQUENCES: 7

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: TO BE ASSIGNED
(B) FILING DATE: 23-DEC-1997
(C) CLASSIFICATION: UNKNOWN

(vii) PRIOR APPLICATION DATA:
(A) APPLICATION NUMBER: 60/051,446
(B) FILING DATE: 01-JUL-1997

(viii) ATTORNEY/AGENT INFORMATION:
(A) NAME: CONNELL, Anthony Christopher
(B) REGISTRATION NUMBER: 5630
(C) REFERENCE/DOCKET NUMBER: GH-70124

(ix) TELECOMMUNICATION INFORMATION:

(A) TELEPHONE: +44 1279 644 395
(B) TELEFAX: +44 181 975 6294
(C) TELEX: 846169

(2) INFORMATION FOR SEQ ID NO:1:

(i) SEQUENCE CHARACTERISTICS:
(A) LENGTH: 2512 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

CCCGAAGGTT GCATGATGGA ATTTGAACAT TACTTCAAGA GGTTTTGTAT TTTGGATTAG 60
TTAATTGGGT TTGTCCTCTG CTGACTGTTT CTTCGGATGC ATTTTTTGGT GTGCTCTTGA 120
GGGATTAAAT GCAAAGAGAT CACACCATGG ACTACAAGGA AAGCTGCCCA AGTGTAAGCA 180
TTCCCAGCTC CGATGAACAC AGAGAGAAAA AGAAGAGGTT TACTGTTTAT AAAGTTCTGG 240
TTTCAGTGGG AAGAAGTGAA TGGTTTGTCT TCAGGAGATA TGCAGAGTTT GATAAACTTT 300
ATAACACTTT AAAAAAACAG TTTCCTGCTA TGGCCCTGAA GATTCCTGCC AAGAGAATAT 360
TTGGTGATAA TTTTGATCCA GATTTTATTA AACAAAGACG AGCAGGACTA AACGAATTCA 420
TTCAGAACCT AGTTAGGTAT CCAGAACTTT ATAAACATCC AGATGTCAGA GCATTCCTTC 480
AAATGGACAG TCCAAAACAC CAGTCAGATC CATCTGAAGA TGAGGATGAA AGAAGTTCTC 540
AGAAGCTACA CTCTACCTCA CAGAACATCA ACCTGGGACC GTCTGGAAAT CCTCATGCCA 600
AACCAACTGA CTTTGATTTC TTAAAAGTTA TTGGAAAAGG CAGCTTTGGC AAGGTTCTTC 660
TTGCAAAACG GAAACTGGAT GGAAAATTTT ATGCTGTCAA AGTGTTACAG AAAAAAATAG 720
TTCTCAACAG AAAAGAGCAA AAACATATTA TGGCTGAACG TAATGTGCTC TTGAAAAATG 780
TGAAACATCC GTTTTTGGTT GGATTGCATT ATTCCTTCCA AACAACTGAA AAGCTTTATT 840
TTGTTCTGGA TTTTGTTAAT GGAGGGGAGC TTTTTTTCCA CTTACAAAGA GAACGGTCCT 900
TTCCTGAGCA CAGAGCTAGG TTTTACGCTG CTGAAATTGC TAGTGCATTG GGTTACTTAC 960
ATTCCATCAA AATAGTATAC AGAGACTTGA AACCAGAAAA TATTCTTTTG GATTCAGTAG 1020
GACATGTTGT CTTAACAGAT TTTGGGCTTT GTAAAGAAGG AATTGCTATT TCTGACACCA 1080
CTACCACATT TTGTGGGACA CCAGAGTATC TTGCACCTGA AGTAATTAGA AAACAGCCCT 1140
ATGACAATAC TGTAGATTGG TGGTGCCTTG GGGCTGTTCT GTATGAAATG CTGTATGGAT 1200
TGCCTCCTTT TTATTGCCGA GATGTTGCTG AAATGTATGA CAATATCCTT CACAAACCCC 1260
TAAGTTTGAG GCCAGGAGTG AGTCTTACAG CCTGGTCCAT TCTGGAAGAA CTCCTAGAAA 1320
AAGACAGGCA AAATCGACTT GGTGCCAAGG AAGACTTTCT TGAAATTCAG AATCATCCTT 1380
TTTTTGAATC ACTCAGCTGG GCTGACCTTG TACAAAAGAA GATTCCACCA CCATTTAATC 1440
CTAATGTGGC TGGACCAGAT GATATCAGAA ACTTTGACAC AGCATTTACA GAAGAAACAG 1500
TTCCATATTC TGTGTGTGTA TCTTCTGACT ATTCTATAGT GAATGCCAGT GTATTGGAGG 1560

CAGATGATGC ATTCGTTGGT TTCTCTTATG CACCTCCTTC AGAAGACTTA TTTTTGTGAG 1620
CAGTTTGCCA TTCAGAAACC ATTGAGCAAA ATAAGTCTAT AGATGGGACT GAAACTTCTA 1680
TTTGTGTGAA TATATTCAAA TATGTATAAC TAGTGCCTCA TTTTTATATG TAATGATGAA 1740
AACTATGAAA AAATGTATTT TCTTCTATGT CCAAGAAAAA TAGGGCATTT CAAAGAGCTG 1800
TTTTGATTAA AATTTATATT CTTGTTTAAT AAGCTTATTT TTAAACAATT TAAAAGCTAT 1860
TATTCTTAGC ATTAACCTAT TTTTAAAGAA ACCTTTTTTG CTATTGACTG TTTTTTCCCT 1920
CTAAGTTTAC ACTAACATCT ACCCAAGATA GACTGTTTTT TAACAGTCAA TTTCAGTTCA 1980
GCTAACATAT ATTAATACCT TTGTAACTCT TTGCTATGGC TTTTGTTATC ACACCAAAAC 2040
TATGCAATTG GTACATGGTT GTTTAAGAAG AAACCGTATT TTTCCATGAT AAATCACTGT 2100
TTGAAATATT TGGTTCATGG TATGATCGAA ATGTAAAAGC ATAATTAACA CATTGGCTGC 2160
TAGTTAACAA TTGGAATAAC TTTATTCTGC AGATCATTTA AGAAGTAACA GGCCGGGCGC 2220
GGTGGCTCAC GCCTGTAATC CCAGCACTTT GGGAGGCTGA GGCGGGCAGA TCACCTGAGG 2280
TCAGGAGTTG GAGACCAGCC TGACCAACAT GGACAAACCC CGTCTCTACT AAAAATACCA 2340
AAATTGGCAG GGTGTGGTGG CACATGCCTA TAATCCCAGC TACTTGGGAG GCTAAGGCAG 2400
GAGAATCGCT TGAACCCGGG AGGCGGAGGT TGCAGTGAGC CGAGATCGCA CCATTGCACT 2460
CCTGCCTGGG CAACAAGAGT GAAACTCCAT CTCCAAAAAA AAAAAAAAAA AA 2512

(2) INFORMATION FOR SEQ ID NO:2:

(i) SEQUENCE CHARACTERISTICS:
(A) LENGTH: 496 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Met Gln Arg Asp His Thr Met Asp Tyr Lys Glu Ser Cys Pro Ser Val
1               5                   10                  15
Ser Ile Pro Ser Ser Asp Glu His Arg Glu Lys Lys Lys Arg Phe Thr
            20                  25                  30
Val Tyr Lys Val Leu Val Ser Val Gly Arg Ser Glu Trp Phe Val Phe
        35                  40                  45
Arg Arg Tyr Ala Glu Phe Asp Lys Leu Tyr Asn Thr Leu Lys Lys Gln
    50                  55                  60
Phe Pro Ala Met Ala Leu Lys Ile Pro Ala Lys Arg Ile Phe Gly Asp
65                  70                  75                  80
Asn Phe Asp Pro Asp Phe Ile Lys Gln Arg Arg Ala Gly Leu Asn Glu
                85                  90                  95
Phe Ile Gln Asn Leu Val Arg Tyr Pro Glu Leu Tyr Lys His Pro Asp
            100                 105                 110

Val Arg Ala Phe Leu Gln Met Asp Ser Pro Lys His Gln Ser Asp Pro
        115                 120                 125
Ser Glu Asp Glu Asp Glu Arg Ser Ser Gln Lys Leu His Ser Thr Ser
    130                 135                 140
Gln Asn Ile Asn Leu Gly Pro Ser Gly Asn Pro His Ala Lys Pro Thr
145                 150                 155                 160
Asp Phe Asp Phe Leu Lys Val Ile Gly Lys Gly Ser Phe Gly Lys Val
                165                 170                 175
Leu Leu Ala Lys Arg Lys Leu Asp Gly Lys Phe Tyr Ala Val Lys Val
            180                 185                 190
Leu Gln Lys Lys Ile Val Leu Asn Arg Lys Glu Gln Lys His Ile Met
        195                 200                 205
Ala Glu Arg Asn Val Leu Leu Lys Asn Val Lys His Pro Phe Leu Val
    210                 215                 220
Gly Leu His Tyr Ser Phe Gln Thr Thr Glu Lys Leu Tyr Phe Val Leu
225                 230                 235                 240
Asp Phe Val Asn Gly Gly Glu Leu Phe Phe His Leu Gln Arg Glu Arg
                245                 250                 255
Ser Phe Pro Glu His Arg Ala Arg Phe Tyr Ala Ala Glu Ile Ala Ser
            260                 265                 270
Ala Leu Gly Tyr Leu His Ser Ile Lys Ile Val Tyr Arg Asp Leu Lys
        275                 280                 285
Pro Glu Asn Ile Leu Leu Asp Ser Val Gly His Val Val Leu Thr Asp
    290                 295                 300
Phe Gly Leu Cys Lys Glu Gly Ile Ala Ile Ser Asp Thr Thr Thr Thr
305                 310                 315                 320
Phe Cys Gly Thr Pro Glu Tyr Leu Ala Pro Glu Val Ile Arg Lys Gln
                325                 330                 335
Pro Tyr Asp Asn Thr Val Asp Trp Trp Cys Leu Gly Ala Val Leu Tyr
            340                 345                 350
Glu Met Leu Tyr Gly Leu Pro Pro Phe Tyr Cys Arg Asp Val Ala Glu
        355                 360                 365
Met Tyr Asp Asn Ile Leu His Lys Pro Leu Ser Leu Arg Pro Gly Val
    370                 375                 380
Ser Leu Thr Ala Trp Ser Ile Leu Glu Glu Leu Leu Glu Lys Asp Arg
385                 390                 395                 400
Gln Asn Arg Leu Gly Ala Lys Glu Asp Phe Leu Glu Ile Gln Asn His
                405                 410                 415
Pro Phe Phe Glu Ser Leu Ser Trp Ala Asp Leu Val Gln Lys Lys Ile
            420                 425                 430
Pro Pro Pro Phe Asn Pro Asn Val Ala Gly Pro Asp Asp Ile Arg Asn
        435                 440                 445
Phe Asp Thr Ala Phe Thr Glu Glu Thr Val Pro Tyr Ser Val Cys Val

450                    455                    460
Ser Ser Asp Tyr Ser Ile Val Asn Ala Ser Val Leu Glu Ala Asp Asp
465                    470                    475                    480
Ala Phe Val Gly Phe Ser Tyr Ala Pro Pro Ser Glu Asp Leu Phe Leu
                485                    490                    495

(2) INFORMATION FOR SEQ ID NO:3:

(i) SEQUENCE CHARACTERISTICS:
(A) LENGTH: 606 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

GAACAGATTT TGGGCTTTGT AAAGAAGGAA TTGCTATTTC TGACACCACT ACCACATTTT 60
GTGGGACACC AGAGTATCTT GCACCTGAAG TAATTAGAAA ACAGCCCTAT GACAATACTG 120
TAGATTGGTG GTGCCTTGGG GCTGTTCTGT ATGAAATGCT GTATGGATTG CCTCCTTTTT 180
ATTGCCGAGA TGTTGCTGAA ATGTATGACA ATATCCTTCA CAAACCCCTA AGTTTGAGGC 240
CAGGAGTGAG TCTTACAGCC TGGTCCATTC TGGAAGAACT CCTAGAAAAA GACAGGCAAA 300
ATCGACTTGG TGCCAAGGAA GACTTTCTTG AAATTCAGAA TCATCCTTTT TTTGAATCAC 360
TCAGCTGGGC TGACCTTGTA CAAAAGAAGA TTCCACCACC ATTTAATCCT AATGTGGCTG 420
GACCAGATGA TATCAGAAAC TTTGACACAG CATTTACAGA AGAAACAGTT CCATATTCTG 480
TGTGTGTATC TTCTGACTAT TCTATAGTGA ATGCCAGTGT ATTGGAGGCA GATGATGCAT 540
TCGTTGGTTT CTCTTATGCA CCTCCTTCAG AAGACTTATT TTTGTGAGCA GTTTGCCATT 600
NCAGGT 606

(2) INFORMATION FOR SEQ ID NO:4:

(i) SEQUENCE CHARACTERISTICS:
(A) LENGTH: 194 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Thr Asp Phe Gly Leu Cys Lys Glu Gly Ile Ala Ile Ser Asp Thr Thr

1                   5                   10                  15
Thr Thr Phe Cys Gly Thr Pro Glu Tyr Leu Ala Pro Glu Val Ile Arg
            20                  25                  30
Lys Gln Pro Tyr Asp Asn Thr Val Asp Trp Trp Cys Leu Gly Ala Val
        35                  40                  45
Leu Tyr Glu Met Leu Tyr Gly Leu Pro Pro Phe Tyr Cys Arg Asp Val
    50                  55                  60
Ala Glu Met Tyr Asp Asn Ile Leu His Lys Pro Leu Ser Leu Arg Pro
65                  70                  75                  80
Gly Val Ser Leu Thr Ala Trp Ser Ile Leu Glu Glu Leu Leu Glu Lys
                85                  90                  95
Asp Arg Gln Asn Arg Leu Gly Ala Lys Glu Asp Phe Leu Glu Ile Gln
            100                 105                 110
Asn His Pro Phe Phe Glu Ser Leu Ser Trp Ala Asp Leu Val Gln Lys
        115                 120                 125
Lys Ile Pro Pro Pro Phe Asn Pro Asn Val Ala Gly Pro Asp Asp Ile
    130                 135                 140
Arg Asn Phe Asp Thr Ala Phe Thr Glu Glu Thr Val Pro Tyr Ser Val
145                 150                 155                 160
Cys Val Ser Ser Asp Tyr Ser Ile Val Asn Ala Ser Val Leu Glu Ala
                165                 170                 175
Asp Asp Ala Phe Val Gly Phe Ser Tyr Ala Pro Pro Ser Glu Asp Leu
            180                 185                 190
Phe Leu

(2) INFORMATION FOR SEQ ID NO:5:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

TGTGGTAGTG GTGTCAGAAT AGCAATTCC                                    29

(2) INFORMATION FOR SEQ ID NO:6:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

AACCTAGCTC TGTGCTCAGG AAAGGAC 27

(2) INFORMATION FOR SEQ ID NO:7:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:7:

TTCCATCCCA GTTTCCGTTT TGCAAGAA 28